# EUROPÄISCHE PATENTSCHRIFT

(11) **EP 0 499 700 B1**
(45) Veröffentlichungstag und Bekanntmachung des Hinweises auf die Patenterteilung: **19.07.1995**
(21) Anmeldenummer: 91120750.4
(22) Anmeldetag: 03.12.1991
(51) Int. Cl.: C07C 69/33, C07C 67/08, C11D 1/66

(54) **Polyglycerinfettsäureestergemisch**
Mixture of fatty acid esters of polyglycerine
Mélange d'esters d'acides gras et de polyglycérine

(30) Priorität: 20.02.1991 DE 4105305
(43) Veröffentlichungstag der Anmeldung: 26.08.1992
(73) Patentinhaber: Deutsche Solvay-Werke Gesellschaft mit beschränkter Haftung, 30173 Hannover (DE)
(72) Erfinder: Jakobson, Gerald, Dr., W-4134 Rheinberg (DE); Siemanowski, Werner, Dr., W-4134 Rheinberg (DE); Uhlig, Karl-Heinz, W-4150 Krefeld-Traar (DE)
(74) Vertreter: Lauer, Dieter, Dr.

(56) Entgegenhaltungen:
- EP-A- 0 299 295
- EP-A- 0 451 461

## Beschreibung

Die Vorliegende Erfindung betrifft ein Polyglycerinfettsäureestergemisch mit ganz bestimmter Zusammensetzung, Verteilung der Fettsäureester und Kettenlängen in der Estergruppe, ein Verdickungsmittel, Hautpflegemittel, Wasch-, Reinigungs- und/oder Körperreinigungsmittel enthaltend dieses Polyglycerinfettsäureestergemisch sowie ein Verfahren zur Herstellung des Polyglycerinfettsäureestergemisches.

Aus der DE-OS 39 02 374 ist bereits ein Wasch-, Reinigungs- und/oder Körperreinigungsmittel bekannt, das neben einem ionogenen und/oder amphoteren Tensid Monofettsäureester mit C₈ bis C₁₈ des Diglycerins und/oder Difettsäureester mit C₈ bis C₁₈ des Tetraglycerins enthält. Dieses Wasch-, Reinigungs- und/oder Körperreinigungsmittel hat eine gute Verarbeitbarkeit, ist hautverträglich und biologisch leicht abbaubar.

Es hat jedoch den Nachteil, daß es in einem relativ teueren und arbeitsaufwendigen Mehrstufenprozeß hergestellt werden muß und normalerweise als festes Produkt anfällt. Bei der Weiterverarbeitung des Produktes, z.B. in Zubereitungen, usw. treten verarbeitungstechnische Schwierigkeiten auf, so z.B. daß das Produkt nicht bei Raumtemperatur gepumpt werden kann. Es besteht das Risiko der Verstopfung von Zuleitungs- und Dosiervorrichtungen. Zur Auflösung des Produktes sind entweder Wärmezufuhr und/oder starke Scherkräfte erforderlich.

Aus der DE-PS 30 41 073 sind weiterhin Wollwachssubstitute auf der Basis von Estergemischen bekannt, bestestehend aus 1 Mol Glycerin-Polyglycerin-Gemisch, verestert mit 0,5 bis 1,1 Mol einer oder mehrerer gesättigter aliphatischer Monocarbonsäuren mit 6 bis 10 C-Atomen, 0,5 bis 1,1 Mol einer oder mehrerer gesättigter aliphatischer Monocarbonsäuren mit 16 bis 22 C-Atomen, 0,0 bis 0,6 Mol einer oder mehrerer aliphatischer gesättigter verzweigtkettiger Monocarbonsäuren mit 16 bis 18 C-Atomen, 0,0 bis 0,5 Mol einer oder mehrerer gesättigter Hydroxymonocarbonsäuren mit 16 bis 20 C-Atomen und 0,5 bis 1,0 Mol einer oder mehrerer gesättigter aliphatischer Dicarbonsäuren mit 4 bis 10 C-Atomen.

Diese Gemische werden unter anderem unter Mitverwendung von Dicarbonsäuren hergestellt, so daß quervernetzte wasserunlösliche Verbindungen entstehen, die somit andere chemische und physikalische Eigenschaften aufweisen.

Ziel und Aufgabe der vorliegenden Erfindung war es, ein Polyglycerinfettsäureestergemisch zu finden, das in einem einfachen Verfahren kostengünstig hergestellt werden kann und bei Umgebungstemperatur flüssig ist. Es sollte bei Raumtemperatur leicht verarbeitbar oder einarbeitbar sein, eine verdickende, rückfettende und/oder hautpflegende Wirkung aufweisen. Das Mittel sollte toxikologisch und ökologisch möglichst unbedenklich sein.

Erfindungsgemäß wurde festgestellt, daß diesen Zielen und Aufgaben ein Polyglycerinfettsäureestergemisch mit einer Fettsäureesterkomponente von C₈ bis C₂₄ gerecht wird, wobei das Polyglycerinfettsäureestergemisch (bezogen auf 100 Gew.-Teile des Polyglycerinfettsäureestergemisches) aus
20 bis 80 Gew.-% Fettsäuremonoester des Polyglycerins mit einem Anteil von mehr als 40 % von C₁₀ und/oder C₈ in der Esterkomponente (gesättigte Fettsäureesterkomponente) des Polyglycerins und
80 bis 20 Gew.-% Fettsäurediester des Polyglycerins mit einem Anteil von 15 bis 0 Gew.-% Tri- und höhere Ester des Polyglycerins und einem Anteil von mehr als 40 % C₁₀ und/oder C₈ in der Esterkomponente (gesättigte Fettsäureesterkomponente) des Polyglycerins besteht, und keine bis nur geringe Mengen von weniger als 5 Gew.-% an freiem Polyglycerin enthält, und wobei das Polyglycerinfettsäureestergemisch einen Anteil von
20 bis 45 Gew.-% Diglycerinfettsäureester,
30 bis 45 Gew.-% Triglycerinfettsäureester und
50 bis 10 Gew.-% Tetra- und höheren Polyglycerinfettsäureestern
aufweist.

Nach einer bevorzugten Ausführungsform besteht das Polyglycerinfettsäureestergemisch (bezogen auf 100 Gew.-Teile des Polyglycerinfettsäureestergemisches) aus
35 bis 65 Gew.-% Fettsäuremonoester des Polyglycerins mit einem Anteil von 50 % und mehr C₁₀ und/oder C₈ in der Esterkomponente (gesättigte Fettsäureesterkomponente) des Polyglycerins und
65 bis 35 Gew.-% Fettsäurediester des Polyglycerins mit einem Anteil von 7 bis 0,5 Gew.-% Tri- und höhere Ester des Polyglycerins und einem Anteil von 50 % und mehr C₁₀ und/oder C₈ in der Esterkomponente (gesättigte Fettsäureesterkomponente) des Polyglycerins, und es enthält keine bis nur geringe Mengen von weniger als 2,5 Gew.-% an freiem Polyglycerin, wobei das Polyglycerinfettsäureestergemisch einen Anteil von
25 bis 42 Gew.-% Diglycerinfettsäureester,
32 bis 43 Gew.-% Triglycerinfettsäureester und
43 bis 15 Gew.-% Tetra- und höheren Polyglycerinfettsäureestern
aufweist.

Bei dem Polyglycerinfettsäureestergemisch aus
20 bis 80 Gew.-%, vorzugsweise 35 bis 65 Gew.-%, Fettsäuremonoester des Polyglycerins mit einem Anteil von mehr als 40 %, vorzugsweise mehr als 50 %, von C₁₀ und/oder C₈ in der Esterkomponente des Polyglycerins sind nach einer Ausführungsform 0 bis 45 %, vorzugsweise 0 bis 30 %, Fettsäuremonoester mit C₁₂ bis C₁₈ in der gesättigten Fettsäureesterkomponente und/oder Fettsäuremonoester mit C₁₂ bis C₂₄ in der ungesättigten Fettsäurekomponente enthalten
und bei den 80 bis 20 Gew.-%, vorzugsweise 65 bis 35 Gew.-%, Fettsäurediester des Polyglycerins mit einem Anteil von 15 bis 0 Gew.-%, vorzugsweise 7 bis 0,5 Gew.-%, Tri- und höhere Ester des Polyglycerins und einem Anteil von mehr als 40 %, vorzugsweise mehr als 50 %, C₁₀ und/oder C₈ in der Esterkomponente des Polyglycerins sind nach dieser Ausführungsform 0 bis 45 %, vorzugsweise 0 bis 30 %, Fettsäurediester mit je C₁₂ bis C₁₈ in der gesättigten Fettsäurekomponente und/oder Fettsäurediester mit C₁₂ bis C₂₄ in der ungesättigten Fettsäureesterkomponente enthalten, und es enthält keine bis nur geringe Mengen von weniger als 5 Gew.-%, vorzugweise von weniger als 2,5 Gew.-%, an freiem Polyglycerin, wobei das Polyglycerinfettsäureestergemisch einen Anteil von
20 bis 45 Gew.-% Diglycerinfettsäureester,
30 bis 45 Gew.-% Triglycerinfettsäureester und
50 bis 10 Gew.-% Tetra- und höheren Polyglycerinfettsäureestern
aufweist.

Nach einer bevorzugten Ausführungsform enthalten jedoch die Fettsäureester des Polyglycerins einen Anteil von mehr als 90 % (oder 90 Gew.-%), vorzugsweise mehr als 94 % (oder 94 Gew.-%) C₁₀ und/oder C₈ in der Estergruppe des Polyglycerins.

Die vorliegende Erfindung betrifft weiterhin ein Verdickungsmittel und/oder Hautpflegeadditiv enthaltend ein Polyglycerinfettsäureestergemisch mit einer Fettsäurekomponente von C₈ bis C₂₄. Das Verdickungsmittel und/oder Hautpflegeadditiv enthält das erfindungsgemäße Polyglycerinfettsäureestergemisch, das (bezogen auf 100 Gew.-Teile des Polyglycerinfettsäureestergemisches) aus
20 bis 80 Gew.-% Fettsäuremonoester des Polyglycerins mit einem Anteil von mehr als 40 % von C₁₀ und/oder C₈ in der Esterkomponente des Polyglycerins und
80 bis 20 Gew.-% Fettsäurediester des Polyglycerins mit einem Anteil von 15 bis 0 Gew.% Tri- und höhere Ester des Polyglycerins und einem Anteil von mehr als 40 % C₁₀ und/oder C₈ in der Esterkomponente des Polyglycerins besteht, und keine bis nur geringe Mengen von weniger als 5 Gew.-% an freiem Polyglycerin enthält, und wobei das Polyglycerinfettsäureestergemisch einen Anteil von
20 bis 45 Gew.-% Diglycerinfettsäureester,
30 bis 45 Gew.-% Triglycerinfettsäureester und
50 bis 10 Gew.-% Tetra- und höheren Polyglycerinfettsäureestern
aufweist.

Das Polyglycerinfettsäureestergemisch des Verdickungsmittels und Hautpflegeadditivs besteht nach einer Ausführungsform (bezogen auf 100 Gew.-Teile des Polyglycerinfettsäureestergemisches) aus 35 bis 65 Gew.-% Fettsäuremonoester des Polyglycerins mit einem Anteil von 50 % und mehr C₁₀ und/oder C₈ in der Esterkomponente des Polyglycerins und
65 bis 35 Gew.-% Fettsäurediester des Polyglycerins mit einem Anteil von 7 bis 0,5 Gew.-% Tri- und höhere Ester des Polyglycerins und einem Anteil von 50 % und mehr C₁₀ und/oder C₈ in der Esterkomponente des Polyglycerins, und enthält keine bis nur geringe Mengen von weniger als 2,5 Gew.-% an freiem Polyglycerin, wobei das Polyglycerinfettsäureestergemisch einen Anteil von
25 bis 42 Gew.-% Diglycerinfettsäureester,
32 bis 43 Gew.-% Triglycerinfettsäureester und
43 bis 15 Gew.-% Tetra- und höheren Polyglycerinfettsäureestern
aufweist.

Nach einer bevorzugten Ausführungsform sind in dem Verdickungsmittel und Hautpflegeadditiv die erfindungsgemäßen Fettsäureester des Polyglycerins mit einem Anteil von mehr als 90 % C₁₀ und/oder C₈, vorzugsweise mehr als 94 % C₁₀ und/oder C₈ in der Estergruppe des Polyglycerins enthalten.

Die Erfindung betrifft weiterhin ein Wasch-, Reinigungs- und/oder Körperreinigungsmittel (einschließlich Haarreinigungsmittel), enthaltend ein Polyglycerinfettsäureestergemisch mit einer Fettsäureesterkomponente von C₈ bis C₂₄ und mindestens ein ionogenes und/oder amphoteres Tensid, wobei gemäß der Erfindung das Polyglycerinfettsäureestergemisch (bezogen auf 100 Gew.-Teile des erfindungsgemäßen Polyglycerinfettsäureestergemisches) aus
20 bis 80 Gew.-% Fettsäuremonoester des Polyglycerins mit einem Anteil von mehr als 40 % von C₁₀ und/oder C₈ in der Esterkomponente des Polyglycerins und
80 bis 20 Gew.-% Fettsäurediester des Polyglycerins mit einem Anteil von 15 bis 0 Gew.-% Tri- und höhere Ester des Polyglycerins und einem Anteil von mehr als 40 % C₁₀ und/oder C₈ in der Esterkomponente des Polyglycerins besteht, und keine bis nur geringe Mengen von weniger als 5 Gew.-% an freiem Polyglycerin enthält, und wobei das Polyglycerinfettsäureestergemisch einen Anteil von
20 bis 45 Gew.-% Diglycerinfettsäureester,
30 bis 45 Gew.-% Triglycerinfettsäureester und
50 bis 10 Gew.-% Tetra- und höheren Polyglycerinfettsäureestern
aufweist.

Wie bereits erwähnt enthält das Wasch-, Reinigungs- und/oder Körperreinigungsmittel (einschließlich Haarreinigungsmittel) das erfindungsgemäße Polyglycerinfettsäureestergemisch, das (bezogen auf 100 Gew.-Teile des Polyglycerinfettsäureestergemisches) aus
35 bis 65 Gew.-% Fettsäuremonoester des Polyglycerins mit einem Anteil von 50 % und mehr C₁₀ und/oder C₈ in der Esterkomponente des Polyglycerins und
65 bis 35 Gew.-% Fettsäurediester des Polyglycerins mit einem Anteil von 7 bis 0,5 Gew.-% Tri- und höhere Ester des Polyglycerins und einem Anteil von 50 % und mehr C₁₀ und/oder C₈ in der Esterkomponente des Polyglycerins besteht, und keine bis nur geringe Mengen von weniger als 2,5 Gew.-% an freiem Polyglycerin enthält. wobei das Polyglycerinfettsäureestergemisch einen Anteil von
25 bis 45 Gew.-% Diglycerinfettsäureester,
32 bis 43 Gew.-% Triglycerinfettsäureester und
43 bis 15 Gew.-% Tetra- und höheren Polyglycerinfettsäureestern
aufweist.

Das erfindungsgemäße Polyglycerinfettsäureestergemisch ist ein Tensid und hat als solches neben anderen Funktionen auch oberflächenaktive Eigenschaften.

Bevorzugt enthält das Wasch-, Reinigungs- und/oder Körperreinigungsmittel (einschließlich Haarreinigungsmittel) die Fettsäureester des Polyglycerins mit einem Anteil von mehr als 90 % C₁₀ und/oder C₈, vorzugsweise von mehr als 94 Gew.-% C₁₀ und/oder C₈, in der Estergruppe des Polyglycerins.

Es war nicht zu erwarten, daß das erfindungsgemäße Polyglycerinfettsäureestergemisch die gewünschten Ergebnisse erbringt und u.a. diese Verbindung auch den gestellten Anforderungen, z.B. dermatologischen sowie toxikologischen Anforderungen hinsichtlich der Wirksamkeit entspricht. Das Wasch-, Reinigungs- und/oder Körperreinigungsmittel enthält bevorzugt zusätzlich Elektrolyte, vorzugsweise anorganische Chloride, in Gewichtsmengen von 0,1 bis 8 Gew.-%, vorzugsweise 0,7 bis 3 Gew.-%, bezogen auf das Gesamtgewicht des Wasch-, Reinigungs- und/oder Körperreinigungsmittels.

Der Gesamttensidgehalt des Mittels unter Verwendung des erfindungsgemäßen Polyglycerinfettsäureestergemisches beträgt 2,5 bis 60 Gew.-%, vorzugsweise 7 bis 50 Gew.-%, und der Restbestandteil besteht aus Lösungs- und/oder Verdünnungsmitteln, vorzugsweise Wasser, niedrigen Alkoholen mit C₁ bis C₈, vorzugsweise C₂ bis C₄, und/oder Glycerin, ausgenommen mehr als 5 Gew.-% Polyglycerin, oder Glycerinderivaten und mindestens einem Elektrolyten sowie ggf. Zusatzmitteln, vorzugsweise Konservierungs-, Parfümierungs-, Färbemitteln, pharmazeutischen Wirkstoffen, Stellmitteln zur pH-Regulierung, Komplexierungsmitteln zur Maskierung von Metallionen, Hautpflegemitteln und/oder Verdickungsmitteln oder enthält diese.

Nach einer bevorzugten Ausführungsform beträgt der Gesamttensidgehalt des Mittels (der Formulierung der Wasch-, Reinigungs- oder Körperreinigungsmittel) 2,5 bis 60 Gew.-%, vorzugsweise 7 bis 50 Gew.-%, worin 0,2 bis 60 Gew.-%, vorzugsweise 1,5 bis 45 Gew.-%, des erfindungsgemäßen Polyglycerinfettsäureestergemisches enthalten sind. Das Mittel enthält 7 bis 0,1 Gew.-%, vorzugsweise 3 bis 0,3 Gew.-%, mindestens eines Elektrolyten, vorzugsweise bestehend aus oder enthaltend Natriumchlorid, sowie 90,5 bis 39,9 Gew.-%, vorzugsweise 90 bis 49,7 Gew.-%, eines Lösungs- und/oder Verdünnungsmittels, vorzugsweise Wasser, niedrige Alkohole mit C₁ bis C₈, vorzugsweise C₂ bis C₄, ausgenommen mehr als 5 Gew.-% Polyglycerin. Es enthält zusätzlich Zusatzmittel in Gewichtsmengen von 0,005 bis 12 Gew.-Teile (bezogen auf 100 Gew.-Teile des aus dem Tensidgemisch (Gesamttensidgemisch), Elektrolyten und Lösungs- und/oder Verdünnungsmittel bestehenden Wasch-, Reinigungs- und/oder Körperreinigungsmittels), vorzugsweise 0,1 bis 5 Gew.-Teile, vorzugsweise bestehend aus Konservierungs-, Parfümierungs-, Färbemitteln, pharmazeutischen Wirkstoffen, Stellmitteln zur pH-Regulierung, Komplexierungsmittel zur Maskierung von Metallionen, Hautpflegemitteln und/oder Verdickungsmitteln.

Das erfindungsgemäße Polyglycerinfettsäureestergemisch erbringt in dem Tensidgemisch für das Körperreinigungsmittel neben milderer Reinigungswirkung bestimmte zusätzliche Eigenschaften, wie z.B. einen Rückfettungseffekt und ein angenehmes Hautgefühl während und nach dem Reinigungsvorgang, ein verbessertes Fließverhalten und weist darüber hinaus eine dermatologische und toxikologische Unbedenklichkeit auf.

Nach einer bevorzugten Ausführungsform enthält das Wasch-, Reinigungs- und/oder Körperreinigungsmittel mindestens ein ionogenes Tensid, bestehend aus mindestens einem Alkylaryl- oder Alkylethersulfat, Alkylhydroxyethersulfonat, Alkylsulfat, Alkylarylsulfonat, vorzugsweise Alkylbenzolsulfonat, Acylaminopolyglykolether-Sulfat, Oleinsulfonat, Paraffinsulfonat, Sulfo-Bernsteinsäureester und/oder Fettalkoholether-Carboxylat und/oder als amphoteres Tensid Alkylamido-Betain und/oder ein amphoteres Glycerinderivat.

Nach einer weiteren bevorzugten Ausführungsform enthält das Wasch-, Reinigungs- und/oder Körperreingungsmittel ein Alkylethersulfat, Alkylethersulfonat, Alkylhydroxyethersulfonat, Aralkylethersulfonat und/oder Alkylsulfat. Als Kation enthalten die Tenside Na⁺, K⁺, Mg⁺⁺, NH4⁺, Alkylammonium, Alkanolammonium, vorzugsweise Monoethanolammonium, Triethanolammonium Na⁺ und/oder NH4⁺.

Nach einer bevorzugten Ausführungsform hat das Wasch-, Reinigungs- und/oder Körperreinigungsmittel einen Gesamttensidgehalt von 2,5 bis 60 Gew.-%, vorzugsweise 7 bis 35 Gew.-%, (bezogen auf 100 Gew.-% des Mittels), wobei das Tensidgemisch 2 bis 30 Gew.-%, vorzugsweise 10 bis 20 Gew.-%, des erfindungsgemäßen Polyglycerinfettsäureestergemisches bezogen auf den Gesamttensidgehalt (100 Gew.-%) und 98 bis 70 Gew.-%, vorzugsweise 90 bis 80 Gew.-%, mindestens eines ionogenen Tensides, bestehend aus mindestens einem Alkylaryl- oder Alkylethersulfat, Alkylsulfat, Alkylethersulfonat, Alkylhydroxyethersulfonat, Polyhydroxyalkylethersulfonat, Alkylarylsulfonat, vorzugsweise Alkylbenzolsulfonat, Acylaminopolyglykolether-Sulfat, Oleinsulfonat, Paraffinsulfonat, Sulfo-Bernsteinsäureester und/oder Fettalkoholether-Carboxylat und/oder als amphoteres Tensid Alkylamido-Betain und/oder amphoteres Glycerinderivat, vorzugsweise jedoch ein ionogenes Tensid oder Tensidgemisch enthaltend oder bestehend aus Alkylethersulfat, Alkylhydroxyethersulfonat, Alkylsulfonat und/oder Alkylsulfat, das als Kation Na⁺, K⁺, Mg⁺⁺, NH4⁺, Monoethanolammonium und/oder Triethanolammonium enthält, und der Restbestandteil aus Lösungs- und/oder Verdünnungsmitteln, vorzugsweise Wasser, niedrige Alkohole mit C₁ bis C₈, vorzugsweise C₂ bis C₄, und/oder Glycerin oder Glycerinderivate, Elektrolyte sowie ggf. Zusatzmittel, vorzugsweise Konservierungs-, Parfümierungs-, Färbemittel, pharmazeutische Wirkstoffe, Stellmittel zur pH-Regulierung, Komplexierungsmittel zur Maskierung von Metallionen, Hautpflegemittel und/oder Verdickungsmittel besteht oder diese enthält.

Nach einer weiteren bevorzugten Ausführungsform enthält dieses Mittel 7 bis 0,1 Gew.-%, vorzugsweise 3 bis 0,3 Gew.-%, mindestens eines Elektrolyten, vorzugsweise bestehend aus oder enthaltend Natriumchlorid und/oder Ammoniumchlorid, 90,5 bis 39,9 Gew.-%, vorzugsweise 90 bis 64,7 Gew.-%, eines Lösungs- und/oder Verdünnungsmittels, vorzugsweise Wasser, niedrige Alkohole mit C₁ bis C₈, vorzugsweise C₂ bis C₄, und/oder Diole mit C₃ bis C₈, vorzugsweise C₃ bis C₆, und Zusatzmittel in Gewichtsmengen von 0,005 bis 12 Gew.-Teile (bezogen auf 100 Gew.-Teile des aus dem Tensidgemisch, Elektrolyten und Lösungs- und/oder Verdünnungsmittels bestehenden Wasch-, Reinigungs- und/oder Körperreinigungsmittel), vorzugsweise 0,1 bis 5 Gew.-Teile, vorzugsweise bestehend aus Konservierungs-, Parfümierungs-, Färbemitteln, pharmazeutischen Wirkstoffen, Stellmitteln zur pH-Regulierung, Komplexierungsmittel zur Maskierung von Metallionen, Hautpflegemitteln und/oder Verdickungsmitteln.

Als Alkylethersulfate, Alkylarylsulfonate, Alkylsulfate und Alkylsulfonate, deren Ester und Olefinsulfonate werden bevorzugt solche mit einer C-Zahl von 8 bis 22, vorzugsweise 10 bis 18, eingesetzt. Bei diesen mit einem hydrophoben Rest in Form eines gesättigten, ungesättigten oder verzweigten Kohlenwasserstoffrest versehenen Tensiden kann der hydrophobe Rest auch über einen Phenylrest und/oder über andere Heteroatome, z.B. Sauerstoff mit der Sulfat- und/oder Sulfonatgruppe verbunden sein, z.B. als Alkylethersulfate. Bevorzugt werden Alkali-, insbesondere Natriumlaurylethersulfat, Acylaminopolyglykolethersulfat, vorzugsweise in Form der Alkanolaminverbindung, insbesondere Triethanolaminsalz, Sulfo-Bernsteinsäureester oder Sulfodicarbonsäureester, Alkali-Benzolsulfonat und Ammoniumalkylbenzolsulfonat, vorzugsweise Natrium- oder Ammoniumalkylbenzolsulfonat oder Triethanolammonium-Alkylbenzolsulfonat und dergleichen eingesetzt.

Als Alkylethersulfate werden bevorzugt Verbindungen der allgemeinen Formel RO(C₂H₄O)ₙSO₃M, in der R eine Alkylkette mit 10 bis 18, vorzugsweise 12 bis 14, Kohlenstoffatomen, n Zahlen von 1 bis 10 und M ein Kation darstellen, eingesetzt. Diese Alkylethersulfate werden durch Ethoxylierung einwertiger Alkohole mit 10 bis 18 Kohlenstoffatomen bis zum gewünschten Ethoxylierungsgrad und nachfolgender Sulfatierung und/oder Neutralisation gewonnen. Die Neutralisation der sauren Sulfatierungsprodukte oder der Sulfonate kann durch Alkalien, Ammoniak, Amine oder Alkanolamine und/oder durch Magnesiumhydroxid erfolgen. Als Alkylhydroxyethersulfonat oder Polyhydroxyalkylethersulfat werden bevorzugt solche mit C₁₀ bis C₁₈, vorzugsweise C₁₂ bis C₁₄, in der Fettsäurekette eingesetzt.

Als Elektrolyte können neben Natrium- und/oder Ammoniumchlorid auch an sich in Körperreinigungsmitteln eingesetzte Elektrolyte zum Einsatz gelangen.

Weiterhin können die Wasch-, Reinigungs- und/oder Körperreinigungsmittel andere Verbindungen wie Kolloide, wie Derivate der Cellulose oder Stärke, desinfizierende Wirkstoffe, fungizide oder antibakterielle Wirkstoffe, Korrosionsschutzmittel usw. enthalten.

Die Erfindung betrifft weiterhin die Verwendung des Wasch-, Reinigungs- und/oder Körperreinigungsmittels als Duschgel oder Duschmittel, Schaumbademittel, flüssiges Handreinigungsmittel oder Haarshampoo.

Die Erfindung betrifft weiterhin ein Verfahren zur Herstellung der erfindungsgemäßen Polyglycerinfettsäureester-Gemische aus Fettsäuren mit C₈ bis C₂₄ und Polyglycerin unter saurer Katalyse. Gemäß der Erfindung wird als Polyglycerin ein Gemisch eingesetzt, das (bezogen auf 100 Gew.-Teile eingesetzter Polyglycerine) aus
20 bis 45 Gew.-% Diglycerin,
30 bis 45 Gew.-% Triglycerin und
50 bis 10 Gew.-% Tetra- und höheren Polyglycerinen
besteht. Dieses Gemisch wird mit der Fettsäure oder dem Fettsäuregemisch
bei 140 bis 200 °C, vorzugsweise
150 bis 160 °C,
in Gegenwart eines sauren Katalysators, vorzugsweise einer sulfonsäuregruppenhaltigen Verbindung, in einem Verhältnis des Polyglyceringemisches zu der Fettsäure oder dem Fettsäuregemisch von
0,5 : 1,5 bis 1,5 : 0,5, vorzugsweise
0,6 : 1 bis 1,2 : 1,
in Gegenwart eines inerten Gases, vorzugsweise Stickstoffes, im Vakuum umgesetzt und das entstehende Reaktionsprodukt kontinuierlich abdestilliert, bis eine Säurezahl von < 3 in dem Reaktionsgemisch erreicht wird. Das Reaktionsgemisch wird auf 60 bis 115 °C, vorzugsweise 80 bis 110 °C, abgekühlt und eventuell ausscheidende Polyglycerinanteile werden entfernt, wonach die Reaktionsmischung enthaltend nicht umgesetzte Polyglycerinanteile von 0 bis 100 °C, vorzugsweise 20 bis 80 °C, mit einem organischen Lösemittel versetzt und nicht umgesetzte Anteile an Polyglycerin mit nachfolgend zugesetztem Wasser, das entsprechend der zugesetzten Katalysatormenge ein Neutralisationsmittel, vorzugsweise in annähernd äquimolekularen Gewichtsmengen zur Katalysatormenge, enthält, in mindestens einer Extraktionsstufe extrahiert werden. Die abgetrennte organische Phase wird von dem eingesetzten organischen Lösungsmittel und dem Restwassergehalt zur Herstellung des Polyglycerinfettsäureestergemisches durch Destillation abgetrennt.

Nach einer vorzugsweisen Ausführungsform wird das Reaktionsgemisch nach Abkühlung auf 60 bis 115 °C, vorzugsweise 80 bis 110 °C, vor der Extraktion und/oder vor der Weiterverarbeitung mehr als 0,5 h, vorzugsweise 1 bis 10 h, stehengelassen und gegebenenfalls ausgeschiedene Anteile von Polyglycerinen werden abgetrennt.

Gemäß einer weiteren Ausführung wird das Reaktionsgemisch zur Extraktion bzw. Entfernung der restlichen (nicht umgesetzten) Polyglycerinanteile mit einem organisch-chemischen Lösemittel oder Lösemittelgemisch vermischt, das ein Wasseraufnahmevermögen von weniger als 30 Gew.-%, vorzugsweise weniger als 20 Gew.-%, (bezogen auf 100 Gew.-Teile des organisch-chemischen Lösemittels oder Lösemittelgemisches), aufweist und/oder mit Wasser bei der Destillation oder in der Gasphase ein azeotropisches Gemisch bildet. Anschliessend wird das restliche Polyglycerin mit Wasser extrahiert. Bevorzugt erfolgt die Extraktion oder Abtrennung von dem freien Polyglycerin, bis ein Anteil von weniger als 5 Gew.-%, vorzugsweise weniger als 2,5 Gew.-%, an Polyglycerin in den erfindungsgemäß hergestellten Polyglycerinfettsäureestergemisch enthalten ist.

Die Erfindung betrifft auch die Verwendung des Polyglycerinfettsäureestergemisches gemäß der Erfindung zur Solubilisierung oder als Emulgator, vorzugsweise zur Solubilisierung von ätherischen Ölen und Parfümölen in Wasser.

Das erfindungsgemäße Polyglycerinfettsäureestergemisch ist vielseitig anwendbar, so z.B. als Verdickungsmittel, Hautpflegeadditiv, Wasch-, Reinigungs-, Körperreinigungsmittel, Badezusatzmittel, als Zusatzmittel für Lebensmittel, insbesondere Fette, Fettersatzstoffe, Süßwaren und Schokoladenwaren; als Zusatzmittel für Arzneimittel, Salben, pharmazeutische Zubereitungen; Zusatzmittel für Desinfektionsmittel, für kosmetische Zubereitungen, für Farben, insbesondere Körperfarben und Farben für Kinder und dergleichen. Die vielseitige Verwendbarkeit ergibt sich u.a. dadurch, daß das erfindungsgemäße Polyglycerinfettsäureestergemisch einerseits leicht verarbeitbar ist, so z.B. Verarbeitung bei Raumtemperatur, wobei die Verarbeitung zahlreicher anderer Produkte mit Hilfe des erfindungsgemäßen Mittels erleichtert wird, andererseits ist das erfindungsgemäße Mittel toxikologisch und ökologisch einwandfrei, haut- und körperneutral bzw. -pflegend und sogar eßbar. Kosmetische Zubereitungen unter Verwendung des erfindungsgemäßen Mittels vermitteln ein optimales pflegendes Gefühl. Bei der Anwendung auf der Haut, z.B. in kosmetischen Zubereitungen, Hautdesinfektionsmitteln, Salben, Einreibungen und dergleichen hat das erfindungsgemäße Polyglycerinfettsäureestergemisch eine rückfettende Wirkung.

### 1. Ausführungsbeispiel

### Polyglycerincaprinat

In einem 2-Liter-Vierhalskolben, der mit Rührwerk, Wasserabscheider, Thermometer und Gaseinleitungsrohr versehen ist, wird eine Mischung von 704 g Polyglycerin (bestehend aus ca. 30 % Diglycerin, ca. 48 % Triglycerin, ca. 13 % Tetraglycerin und ca. 9 % höheren Oligomeren), 688 g Caprinsäure, 2,8 g Dodecylbenzolsulfonsäure und 1,1 g hypophosphorige Säure unter Rühren und gleichzeitigem Durchleiten von Inertgas während ca. 4 Stunden bei 400 bis 40 mbar bis max. 165 °C erhitzt, wobei das Reaktionswasser kontinuierlich destillativ entfernt wird. Bei Erreichen einer Säurezahl von < 2 wird das Reaktionsgemisch auf ca. 60 bis 65 °C heruntergekühlt und nicht umgesetzte Anteile an Polyglycerin wie folgt entfernt:
Das Reaktionsgemisch wird in Ethylacetat (gleicher Volumenanteil) gelöst und dreistufig mit Wasser extrahiert. In der 1. Stufe wird dem Wasser die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt 124 g. Die nach der Extraktion verbleibende organische Phase wird im Vakuum eingedampft.

| **Kennzahlen** | | **Anteile der Ester in Gew.-%** | |
|---|---|---|---|
| Säurezahl: | 0,6 | | |
| Verseifungszahl: | 184,9 | Monoester: | ca. 40 |
| Viskosität bei 25 °C: | 2100 mPa s | Diester: | ca. 50 |
| Farbe nach Gardner: | 2 - 3 | höhere Ester: | ca. 10 |

### 2. Ausführungsbeispiel

### Vorlauffettsäureester des Polyglycerins

In einem 2-Liter-Vierhalskolben, der mit Rührwerk, Wasserabscheider, Thermometer und Gaseinleitungsrohr versehen ist, wird eine Mischung von 704 g Polyglycerin (bestehend aus ca. 30 % Diglycerin, ca. 48 % Triglycerin, ca. 13 % Tetraglycerin und ca. 9 % höheren Oligomeren), 616 g Caprinsäure, 2,8 g Dodecylbenzolsulfonsäure und 1,1 g hypophosphorige Säure unter Rühren und gleichzeitigem Durchleiten von Inertgas während ca. 4 Stunden bei 400 bis 40 mbar bis max. 165 °C erhitzt, wobei das Reaktionswasser kontinuierlich destillativ entfernt wird. Bei Erreichen einer Säurezahl < 2 wird das Reaktionsgemisch auf ca. 60 bis 65 °C heruntergekühlt und nicht umgesetzte Anteile an Polyglycerin wie folgt entfernt:
Das Reaktionsgemisch wird in Ethylacetat (gleicher Volumenanteil) gelöst und dreistufig mit Wasser extrahiert. In der 1. Stufe wird dem Wasser die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt 44 g. Die nach der Extraktion verbleibende organische Phase wird im Vakuum eingedampft.

| **Kennzahlen** | | **Anteile der Ester in Gew.-%** | |
|---|---|---|---|
| Säurezahl: | 0,6 | | |
| Verseifungszahl: | 198,9 | Monoester: | ca. 35 |
| Viskosität bei 25 °C: | 2000 mPa s | Diester: | ca. 55 |
| Farbe nach Gardner: | 1 - 2 | höhere Ester: | ca. 10 |

### 3. Ausführungsbeispiel

### Polyglycerincocoat

In einem 2-Liter Vierhalskolben, der mit Rührwerk, Wasserabscheider, Thermometer und Gaseinleitungsrohr versehen ist, wird eine Mischung von 717 g Polyglycerin (bestehend aus ca. 30 % Diglycerin, ca. 39 % Triglycerin, ca. 19 % Tetraglycerin und ca. 12 % höheren Oligomeren), 856 g Cocosfettsäure, 2,8 g Dodecylbenzolsulfonsäure und 1,1 g hypophosphorige Säure unter Rühren und gleichzeitigem Durchleiten von Inertgas während ca. 4 Stunden bei 400 bis 40 mbar bis max. 165 °C erhitzt, wobei das Reaktionswasser kontinuierlich destillativ entfernt wird. Bei Erreichen einer Säurezahl von < 2 wird das Reaktionsgemisch auf ca. 60 bis 65 °C heruntergekühlt und nicht umgesetzte Anteile an Polyglycerin wie folgt entfernt:
Das Reaktionsgemisch wird in Ethylacetat (gleicher Volumenanteil) gelöst und dreistufig mit Wasser extrahiert. In der 1. Stufe wird dem Wasser die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt 93 g. Die nach der Extraktion verbleibende organische Phase wird im Vakuum eingedampft.

| **Kennzahlen** | | **Anteile der Ester in Gew.-%** | |
|---|---|---|---|
| Säurezahl: | 1,5 | | |
| Verseifungszahl: | 161,5 | Monoester: | ca. 40 |
| Viskosität bei 25 °C: | 5800 mPa s | Diester: | ca. 50 |
| Farbe nach Gardner: | 3 | höhere Ester: | ca. 10 |

### 4. Ausführungsbeispiel

### Polyglycerincaprinat

In einem 2-Liter-Vierhalskolben, der mit Rührwerk, Wasserabscheider, Thermometer und Gaseinleitungsrohr versehen ist, werden zu 896 g Polyglycerin (bestehend aus ca. 30 % Diglycerin, ca. 48 % Triglycerin, ca. 13 % Tetraglycerin und ca. 9 % höheren Oligomeren), 2,8 g Dodecylbenzolsulfonsäure und 1,1 g hypophosphoriger Säure unter Rühren und gleichzeitigem Durchleiten von Inertgas 688 g Caprinsäure innerhalb 3 Stunden kontinuierlich zudosiert (bei einer Temperatur von max. 165 °C und 100 bis 40 mbar), wobei das Reaktionswasser während der gesamten Reaktionszeit destillativ entfernt wird. Bei Erreichen einer Säurezahl von < 2 wird das Reaktionsgemisch auf ca. 60 bis 65 °C heruntergekühlt und nicht umgesetzte Anteile an Polyglycerin wie folgt entfernt:
Das Reaktionsgemisch wird in Ethylacetat (gleicher Volumenanteil) gelöst und dreistufig mit Wasser extrahiert. In der 1. Stufe wird dem Wasser die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt 203 g. Die nach der Extraktion verbleibende organische Phase wird im Vakuum eingedampft.

| **Kennzahlen** | | **Anteile der Ester in Gew.-%** | |
|---|---|---|---|
| Säurezahl: | 0,8 | | |
| Verseifungszahl: | 168,5 | Monoester: | ca. 70 |
| Viskosität bei 25 °C: | 6012 mPa s | Diester: | ca. 25 |
| Farbe nach Gardner: | 2 - 3 | höhere Ester: | ca. 5 |

### 5. Ausführungsbeispiel

### Polyglycerincaprinat

In einem 2-Liter Vierhalskolben, der mit Rührwerk, Wasserabscheider, Thermometer und Gaseinleitungsrohr versehen ist, wird eine Mischung von 896 g Polyglycerin (bestehend aus ca. 30 % Diglycerin, ca. 48 % Triglycerin, ca. 13 % Tetraglycerin und ca. 9 % höheren Oligomeren), 688 g Caprinsäure, 2,8 g Dodecylbenzolsulfonsäure und 1,1 g hypophosphorige Säure unter Rühren und gleichzeitigem Durchleiten von Inertgas während ca. 4 Stunden bei 400 bis 40 mbar bis max. 165 °C erhitzt, wobei das Reaktionswasser kontinuierlich destillativ entfernt wird. Bei Erreichen einer Säurezahl von < 2 wird das Reaktionsgemisch auf ca. 60 bis 65 °C heruntergekühlt und nicht umgesetzte Anteile an Polyglycerin wie folgt entfernt:
Das Reaktionsgemisch wird in Ethylacetat (gleicher Volumenanteil) gelöst und dreistufig mit Wasser extrahiert. In der 1. Stufe wird dem Wasser die der Säurezahl entsprechende Menge an Natronlauge zugesetzt. Die Gesamtmenge des zur Extraktion verwendeten Wassers beträgt ca. 87 Vol.-% des eingesetzten Esters. Die Summe der extrahierten Anteile an Polyglycerin nach Abdestillation der Lösungsmittel beträgt 228 g. Die nach der Extraktion verbleibende organische Phase wird im Vakuum eingedampft.

| **Kennzahlen** | | **Anteile der Ester in Gew.-%** | |
|---|---|---|---|
| Säurezahl: | 1,0 | | |
| Verseifungszahl: | 173,3 | Monoester: | ca. 60 |
| Viskosität bei 25 °C: | 4329 mPa s | Diester: | ca. 32 |
| Farbe nach Gardner: | 2 - 3 | höhere Ester: | ca. 8 |

### 6. Ausführungsbeispiel

6.1. Synthese von Polyglycerincaprinat gemäß Ausführungsbeispiel 1.
6.2. Vergleich der Verdickungseigenschaften des Esters vor bzw. nach der Extraktion nicht umgesetzter Anteile Polyglycerin.
Einer 14 %igen Natriumlaurylethersulfatlösung, welche 2 % Natriumchlorid enthielt, wurde das im 1. Ausführungsbeispiel synthetisierte Polyglycerincaprinat in aufsteigender Menge zugefügt und die resultierende Viskosität gemessen:
- 97 g o.g. Lösung + 3 g Polyglycerincaprinat; daraus resultierende Viskositäten:

| | |
|---|---|
| 4000 mPa s | 7200 mPa s |
| **ohne** Extraktion | **nach** Extraktion. |

### Anwendungsbeispiele

### Schaum- und Verdickungsvermögen

Einer 14 %igen Natriumlaurylethersulfatlösung, welche 2 % Natriumchlorid enthielt, wurde das im 1. Ausführungsbeispiel synthetisierte Polyglycerincaprinat in aufsteigender Menge zugefügt und die resultierende Viskosität gemessen:

| | |
|---|---|
| 1. Lösung ohne Zusatz | 14 mPa s |
| 2. 99 g Lösung + 1 g Polyglycerincaprinat | 187 mPa s |
| 3. 98 g Lösung + 2 g Polyglycerincaprinat | 935 mPa s |
| 4. 97 g Lösung + 3 g Polyglycerincaprinat | 6600 mPa s |

### Schaumbestimmung nach Ross / Miles DIN 53 902 Teil 2 (in einer 1 g/l Aktivsubstanzlösung in destilliertem Wasser bei 40 °C)

| | Schaumentwicklung nach | | |
|---|---|---|---|
| | 30 Sekunden | 3 Minuten | 5 Minuten |
| aus Lösung 1. ohne Zusatz | 156 mm | 159 mm | 158 mm |
| aus Lösung 4. + 3 g Polyglycerincaprinat | 171 mm | 162 mm | 156 mm |

Man erkennt, daß neben einer erheblichen Verdickungswirkung auch eine deutliche Verbesserung des Anschäumvermögens auftritt:
nach 30 Sekunden Steigerung von 156 mm auf 171 mm = 10 %.

### Formulierungen

| **1. Haarshampoo, mild, 12 % Aktivgehalt** | |
|---|---|
| Natriumlaurylethersulfat 28 %ig | 21,4 Gew.-% |
| Cocosamidopropyl-Betain 30 %ig | 6,7 Gew.-% |
| Dinatriumfettalkoholpolyglykolethersulfosuccinat 40 %ig | 5,0 Gew.-% |
| erfindungsgemäßes Polyglycerincaprinat (gemäß Beispiel 4 oder 5) | 2,0 Gew.-% |
| Natriumchlorid | 1,5 Gew.-% |
| Parfüm, Konservierungsmittel, Stellmittel und Wasser | zu 100 Gew.-% |
| Viskosität, gemessen mit Rotationsviskosimeter: | |
| VT 181, Firma Haake, bei 20 °C | 3150 mPa s |
| Schaumzahl nach Ross / Miles (1 g/l Aktivsubstanz bei 40 °C in dest. Wasser) | 176 / 171 / 170 |
| Kälte-Stabilität der Formulierung | < 0 °C |

| **2. Haarshampoo, mild, 16 % Aktivgehalt** | |
|---|---|
| Natriumlaurylethersulfat 28 %ig | 20,0 Gew.-% |
| Cocosamidopropyl-Betain 30 %ig | 8,9 Gew.-% |
| Dinatriumfettalkoholpolyglykolethersulfosuccinat 40 %ig | 13,4 Gew.-% |
| erfindungsgemäßes Polyglycerincaprinat (gemäß Beispiel 1 oder 4) | 2,7 Gew.-% |
| Natriumchlorid | 1,5 Gew.-% |
| Parfüm, Konservierungsmittel, Stellmittel und Wasser | zu 100 Gew.-% |
| Viskosität bei 20 °C | 1400 mPa s |
| Schaumzahl nach Ross / Miles (1 g/l Aktivsubstanz bei 40 °C in dest. Wasser) | 167 / 164 / 161 |
| Kältestabilität der Formulierung | < 0 °C |

| **3. Schaumbad, 20 % Aktivsubstanz** | |
|---|---|
| Natriumlaurylethersulfat 28 %ig | 28,6 Gew.-% |
| Cocosamidopropyl-Betain 30 %ig | 6,7 Gew.-% |
| Dinatriumfettalkoholglykolethersulfosuccinat 40 %ig | 17,5 Gew.-% |
| erfindungsgemäßes Polyglycerincaprinat (gemäß Beispiel 1, 4 oder 5) | 3,0 Gew.-% |
| Natriumchlorid | 1,0 Gew.-% |
| Parfüm, Konservierungsmittel, Stellmittel und Wasser | zu 100 Gew.-% |
| Viskosität bei 20 °C | 1300 mPa s |
| Schaumzahl nach Ross / Miles (1 g/l Aktivsubstanz bei 40 °C in dest. Wasser) | 164 / 159 / 158 |
| Kältestabilität der Formulierung | < 0 °C |

| **4. Flüssigseife, 13,5 % Aktivgehalt** | |
|---|---|
| Natriumlaurylethersulfat 28 %ig | 7,1 Gew.-% |
| Monoethanolaminlaurylsulfat 30 %ig | 12,0 Gew.-% |
| Natriumpolyethylenglykol-6-cocosamidcarboxylat 30 %ig | 15,0 Gew.-% |
| Dinatriumfettalkoholpolyglykolethersulfosuccinat 40 %ig | 5,0 Gew.-% |
| erfindungsgemäßes Polyglycerincaprinat (gemäß Beispiel 4 oder 5) | 1,5 Gew.-% |
| Natriumchlorid | 1,0 Gew.-% |
| Parfüm, Konservierungsmittel, Stellmittel und Wasser | zu 100 Gew.-% |
| Viskosität bei 20 °C | 660 mPa s |
| Schaumzahl nach Ross / Miles (1 g/l Aktivsubstanz bei 40 °C in dest. Wasser) | 167 / 154 / 138 |
| Kältestabilität der Formulierung | < 0 °C |

| **5. Pflegendes Ölbad** | |
|---|---|
| Rosmarinöl | 42 Gew.-% |
| erfindungsgemäßes Polyglycerincaprinat (gemäß Beispiel 2 oder 4) (als Hautpflegeadditiv und Solubilisator) | 43 Gew.-% |
| Wasser, voll entsalzt | 15 Gew.-% |

## Patentansprüche

1. Polyglycerinfettsäureestergemisch mit einer Fettsäureesterkomponente von C₈ bis C₂₄, dadurch gekennzeichnet, daß das Polyglycerinfettsäureestergemisch (bezogen auf 100 Gew.-Teile des Polyglycerinfettsäureestergemisches) aus
20 bis 80 Gew.-% Fettsäuremonoester des Polyglycerins mit einem Anteil von mehr als 40 % von C₁₀ und/oder C₈ in der Esterkomponente des Polyglycerins und
80 bis 20 Gew.-% Fettsäurediester des Polyglycerins mit einem Anteil von 15 bis 0 Gew.-% Tri- und höhere Ester des Polyglycerins und einem Anteil von mehr als 40 % C₁₀ und/oder C₈ in der Esterkomponente des Polyglycerins besteht,
und keine bis nur geringe Mengen von weniger als 5 Gew.-% an freiem Polyglycerin enthält,
wobei das Polyglycerinfettsäureestergemisch einen Anteil von
20 bis 45 Gew.-% Diglycerinfettsäureester,
20 bis 45 Gew.-% Triglycerinfettsäureester und
50 bis 10 Gew.-% Tetra- und höheren Polyglycerinfettsäureestern
aufweist.

2. Polyglycerinfettsäureestergemisch nach Anspruch 1, dadurch gekennzeichnet, daß das Polyglycerinfettsäureestergemisch (bezogen auf 100 Gew.-Teile des Polyglycerinfettsäureestergemisches) aus
35 bis 65 Gew.-% Fettsäuremonoester des Polyglycerins mit einem Anteil von 50 % und mehr C₁₀ und/oder C₈ in der Esterkomponente des Polyglycerins und
65 bis 35 Gew.-% Fettsäurediester des Polyglycerins mit einem Anteil von 7 bis 0,5 Gew.-% Tri- und höhere Ester des Polyglycerins und einem Anteil von 50 % und mehr C₁₀ und/oder C₈ in der Esterkomponente des Polyglycerins besteht,
und keine bis nur geringe Mengen von weniger als 2,5 Gew.-% an freiem Polyglycerin enthält,
wobei das Polyglycerinfettsäureestergemisch einen Anteil von
25 bis 42 Gew.-% Diglycerinfettsäureester,
32 bis 43 Gew.-% Triglycerinfettsäureester und
43 bis 15 Gew.-% Tetra- und höheren Polyglycerinfettsäureestern
aufweist.

3. Polyglycerinfettsäureestergemisch nach Ansprüchen 1 und 2, dadurch gekennzeichnet, daß die Fettsäureester des Polyglycerins einen Anteil von mehr als 90 % C₁₀ und/oder C₈ in der Estergruppe des Polyglycerins enthalten.

4. Verdickungsmittel und/oder Hautpflegeadditiv enthaltend ein Polyglycerinfettsäureestergemisch mit einer Fettsäurekomponente von C₈ bis C₂₄, dadurch gekennzeichnet, daß das Polyglycerinfettsäureestergemisch (bezogen auf 100 Gew.-Teile des Polyglycerinfettsäureestergemisches) aus
20 bis 80 Gew.-% Fettsäuremonoester des Polyglycerins mit einem Anteil von mehr als 40 % von C₁₀ und/oder C₈ in der Esterkomponente des Polyglycerins und
80 bis 20 Gew.-% Fettsäurediester des Polyglycerins mit einem Anteil von 15 bis 0 Gew.-% Tri- und höhere Ester des Polyglycerins und einem Anteil von mehr als 40 % C₁₀ und/oder C₈ in der Esterkomponente des Polyglycerins besteht,
und keine bis nur geringe Mengen von weniger als 5 Gew.-% an freiem Polyglycerin enthält,
wobei das Polyglycerinfettsäureestergemisch einen Anteil von
20 bis 45 Gew.-% Diglycerinfettsäureester,
30 bis 45 Gew.-% Triglycerinfettsäureester und
50 bis 10 Gew.-% Tetra- und höheren Polyglycerinfettsäureestern
aufweist.

5. Verdickungsmittel und/oder Hautpflegeadditiv nach Anspruch 4, dadurch gekennzeichnet, daß das Polyglycerinfettsäureestergemisch (bezogen auf 100 Gew.-Teile des Polyglycerinfettsäureestergemisches) aus
35 bis 65 Gew.-% Fettsäuremonoester des Polyglycerins mit einem Anteil von 50 % und mehr C₁₀ und/oder C₈ in der Esterkomponente des Polyglycerins und
65 bis 35 Gew.-% Fettsäurediester des Polyglycerins mit einem Anteil von 7 bis 0,5 Gew.-% Tri- und höhere Ester des Polyglycerins und einem Anteil von 50 % und mehr C₁₀ und/oder C₈ in der Esterkomponente des Polyglycerins besteht,
und keine bis nur geringe Mengen von weniger als 2,5 Gew.-% an freiem Polyglycerin enthält,
wobei das Polyglycerinfettsäureestergemisch einen Anteil von
25 bis 42 Gew.-% Diglycerinfettsäureester,
32 bis 43 Gew.-% Triglycerinfettsäureester und
43 bis 15 Gew.-% Tetra- und höheren Polyglycerinfettsäureestern
aufweist.

6. Verdickungsmittel und/oder Hautpflegeadditiv nach Ansprüchen 4 und 5, dadurch gekennzeichnet, daß die Fettsäureester des Polyglycerins einen Anteil von mehr als 90 % C₁₀ und/oder C₈ in der Estergruppe des Polyglycerins enthalten.

7. Wasch-, Reinigungs- und/oder Körperreinigungsmittel (einschließlich Haarreinigungsmittel), enthaltend ein Polyglycerinfettsäureestergemisch mit einer Fettsäureesterkomponente von C₈ bis C₂₄ und mindestens ein ionogenes und/oder amphoteres Tensid, dadurch gekennzeichnet, daß das Polyglycerinfettsäureestergemisch (bezogen auf 100 Gew.-Teile des Polyglycerinfettsäureestergemisches) aus
20 bis 80 Gew.-% Fettsäuremonoester des Polyglycerins mit einem Anteil von mehr als 40 % von C₁₀ und/oder C₈ in der Esterkomponente des Polyglycerins und
80 bis 20 Gew.-% Fettsäurediester des Polyglycerins mit einem Anteil von 15 bis 0 Gew.-% Tri- und höhere Ester des Polyglycerins und einem Anteil von mehr als 40 % C₁₀ und/oder C₈ in der Esterkomponente des Polyglycerins besteht,
und keine bis nur geringe Mengen von weniger als 5 Gew.-% an freiem Polyglycerin enthält,
wobei das Polyglycerinfettsäureestergemisch einen Anteil von
20 bis 45 Gew.-% Diglycerinfettsäureester,
30 bis 45 Gew.-% Triglycerinfettsäureester und
50 bis 10 Gew.-% Tetra- und höheren Polyglycerinfettsäureestern
aufweist.

8. Wasch-, Reinigungs- und/oder Körperreinigungsmittel (einschließlich Haarreinigungsmittel) nach Anspruch 7, dadurch gekennzeichnet, daß das Polyglycerinfettsäureestergemisch (bezogen auf 100 Gew.-Teile des Polyglycerinfettsäureestergemisches) aus
35 bis 65 Gew.-% Fettsäuremonoester des Polyglycerins mit einem Anteil von 50 % und mehr C₁₀ und/oder C₈ in der Esterkomponente des Polyglycerins und
65 bis 35 Gew.-% Fettsäurediester des Polyglycerins mit einem Anteil von 7 bis 0,5 Gew.-% Tri- und höhere Ester des Polyglycerins und einem Anteil von 50 % und mehr C₁₀ und/oder C₈ in der Esterkomponente des Polyglycerins besteht,
und keine bis nur geringe Mengen von weniger als 2,5 Gew.-% an freiem Polyglycerin enthält,
wobei das Polyglycerinfettsäureestergemisch einen Anteil von
25 bis 42 Gew.-% Diglycerinfettsäureester,
32 bis 43 Gew.-% Triglycerinfettsäureester und
43 bis 15 Gew.-% Tetra- und höheren Polyglycerinfettsäureestern
aufweist.

9. Wasch-, Reinigungs- und/oder Körperreinigungsmittel (einschließlich Haarreinigungsmittel) nach Ansprüchen 7 und 8, dadurch gekennzeichnet, daß die Fettsäureester des Polyglycerins einen Anteil von mehr als 90 % C₁₀ und/oder C₈ in der Estergruppe des Polyglycerins enthalten.

10. Verfahren zur Herstellung von Polyglycerinfettsäureestern mit einer der in den Ansprüchen 1 bis 3 angegebenen Zusammensetzung aus Fettsäuren mit C₈ bis C₂₄ und Polyglycerin unter saurer Katalyse, dadurch gekennzeichnet, daß als Polyglycerin ein Gemisch eingesetzt wird, das (bezogen auf 100 Gew.-Teile eingesetzter Polyglycerine) aus
20 bis 45 Gew.-% Diglycerin,
30 bis 45 Gew.-% Triglycerin und
50 bis 10 Gew.-% Tetra- und höheren Polyglycerinen
besteht und mit der Fettsäure oder dem Fettsäuregemisch
bei 140 bis 220 °C, vorzugsweise
150 bis 160 °C,
in Gegenwart eines sauren Katalysators, vorzugsweise einer sulfonsäuregruppenhaltigen Verbindung, in einem Verhältnis des Polyglyceringemisches zu der Fettsäure oder dem Fettsäuregemisch von
0,5:1,5 bis 1,5:0,5, vorzugsweise
0,6:1 bis 1,2:1,
in Gegenwart eines inerten Gases, vorzugsweise Stickstoffes, im Vakuum umgesetzt und das entstehende Reaktionsprodukt kontinuierlich abdestilliert wird, bis eine Säurezahl von < 3 in dem Reaktionsgemisch erreicht wird, das Reaktionsgemisch auf 60 bis 115 °C, vorzugsweise 80 bis 110 °C, abgekühlt und eventuell ausscheidende Polyglycerinanteile entfernt werden, wonach die Reaktionsmischung, enthaltend nicht umgesetzte Polyglycerinanteile bei 0 bis 100 °C, vorzugsweise bis 20 bis 80 °C, mit einem organischen Lösemittel versetzt und nicht umgesetzte Anteile an Polyglycerin mit nachfolgend zugesetztem Wasser, das entsprechend der zugesetzten Katalysatormenge ein Neutralisationsmittel, vorzugsweise in annähernd äquimolekularen Gewichtsmengen zur Katalysatormenge, enthält, in mindestens einer Extraktionsstufe extrahiert werden und die abgetrennte organische Phase von dem eingesetzten organischen Lösungsmittel und dem Restwassergehalt zur Herstellung des Polyglycerinfettsäureestergemisches durch Destillation abgetrennt wird.

11. Verfahren nach Anspruch 10, dadurch gekennzeichnet, daß das Reaktionsgemisch nach Abkühlung auf 60 bis 115 °C, vorzugsweise 80 bis 110 °C, vor der Extraktion und/oder vor der Weiterverarbeitung mehr als 0,5 h, vorzugsweise 1 bis 10 h, stehengelassen wird und gegebenenfalls ausgeschiedene Anteile von Polyglycerinen abgetrennt werden.

12. Verfahren nach Ansprüchen 10 und 11, dadurch gekennzeichnet, daß das Reaktionsgemisch zur Extraktion bzw. Entfernung der restlichen (nicht umgesetzten) Polyglycerinanteile mit einem organisch-chemischen Lösemittel oder Lösemittelgemisch vermischt wird, das ein Wasseraufnahmevermögen von weniger als 30 Gew.-%, vorzugsweise weniger als 20 Gew.-% (bezogen auf 100 Gew.-Teile des organisch-chemischen Lösemittels oder Lösemittelgemisches), aufweist und/oder mit Wasser bei der Destillation oder in der Gasphase ein azeotropisches Gemisch bildet und anschließend das restliche Polyglycerin mit Wasser extrahiert wird.

13. Verwendung des Polyglycerinfettsäureestergemisches nach Ansprüchen 1 bis 3 zur Solubilisierung oder als Emulgator, vorzugsweise zur Solubilisierung von ätherischen Ölen und Parfümölen in Wasser.

## Claims

1. A polyglycerol fatty acid ester mixture with a fatty acid ester component of C₈ to C₂₄, characterised in that the polyglycerol fatty acid ester mixture (relative to 100 parts by weight of the polyglycerol fatty acid ester mixture) consists of
20 to 80% by weight fatty acid monoester of the polyglycerol with a content of more than 40% of C₁₀ and/or C₈ in the ester component of the polyglycerol and
80 to 20% by weight fatty acid diester of the polyglycerol with a content of 15 to 0% by weight triesters and higher esters of the polyglycerol and a content of more than 40% C₁₀ and/or C₈ in the ester component of the polyglycerol,
and no quantities to only small quantities of less than 5% by weight of free polyglycerol,
the polyglycerol fatty acid ester mixture having a content of
20 to 45% by weight diglycerol fatty acid ester,
20 to 45% by weight triglycerol fatty acid ester and
50 to 10% by weight tetra- and higher polyglycerol fatty acid esters.

2. A polyglycerol fatty acid ester mixture according to Claim 1, characterised in that the polyglycerol fatty acid ester mixture (relative to 100 parts by weight of the polyglycerol fatty acid ester mixture) consists of
35 to 65% by weight fatty acid monoester of the polyglycerol with a content of 50% and more C₁₀ and/or C₈ in the ester component of the polyglycerol and
65 to 35% by weight fatty acid diester of the polyglycerol with a content of 7 to 0.5% by weight triesters and higher esters of the polyglycerol and a content of 50% and more C₁₀ and/or C₈ in the ester component of the polyglycerol,
and no quantities to only small quantities of less than 2.5% by weight of free polyglycerol,
the polyglycerol fatty acid ester mixture having a content of
25 to 42% by weight diglycerol fatty acid ester,
32 to 43% by weight triglycerol fatty acid ester and
43 to 15% by weight tetra- and higher polyglycerol fatty acid esters.

3. A polyglycerol fatty acid ester mixture according to Claims 1 and 2, characterised in that the fatty acid esters of the polyglycerol have a content of more than 90% C₁₀ and/or C₈ in the ester group of the polyglycerol.

4. A thickening agent and/or skin-care additive containing a polyglycerol fatty acid ester mixture with a fatty acid component of C₈ to C₂₄, characterised in that the polyglycerol fatty acid ester mixture (relative to 100 parts by weight of the polyglycerol fatty acid ester mixture) consists of
20 to 80% by weight fatty acid monoester of the polyglycerol with a content of more than 40% of C₁₀ and/or C₈ in the ester component of the polyglycerol and 80 to 20% by weight fatty acid diester of the polyglycerol with a content of 15 to 0% by weight triesters and higher esters of the polyglycerol and a content of more than 40% C₁₀ and/or C₈ in the ester component of the polyglycerol,
and no quantities to only small quantities of less than 5% by weight of free polyglycerol,
the polyglycerol fatty acid ester mixture having a content of
20 to 45% by weight diglycerol fatty acid ester,
30 to 45% by weight triglycerol fatty acid ester and
50 to 10% by weight tetra- and higher polyglycerol fatty acid esters.

5. A thickening agent and/or skin-care additive according to Claim 4, characterised in that the polyglycerol fatty acid ester mixture (relative to 100 parts by weight of the polyglycerol fatty acid ester mixture) consists of
35 to 65% by weight fatty acid monoester of the polyglycerol with a content of 50% and more C₁₀ and/or C₈ in the ester component of the polyglycerol and
65 to 35% by weight fatty acid diester of the polyglycerol with a content of 7 to 0.5% by weight triesters and higher esters of the polyglycerol and a content of 50% and more C₁₀ and/or C₈ in the ester component of the polyglycerol,
and no quantities to only small quantities of less than 2.5% by weight of free polyglycerol,
the polyglycerol fatty acid ester mixture having a content of
25 to 42% by weight diglycerol fatty acid ester,
32 to 43% by weight triglycerol fatty acid ester and
43 to 15% by weight tetra- and higher polyglycerol fatty acid esters.

6. A thickening agent and/or skin-care additive according to Claims 4 and 5, characterised in that the fatty acid esters of the polyglycerol have a content of more than 90% C₁₀ and/or C₈ in the ester group of the polyglycerol.

7. A detergent, cleaning agent and/or body-cleansing agent (including hair-cleansing agent), containing a polyglycerol fatty acid ester mixture with a fatty acid ester component of C₈ to C₂₄ and at least one ionogenic and/or amphoteric surfactant, characterised in that the polyglycerol fatty acid ester mixture (relative to 100 parts by weight of the polyglycerol fatty acid ester mixture) consists of
20 to 80% by weight fatty acid monoester of the polyglycerol with a content of more than 40% of C₁₀ and/or C₈ in the ester component of the polyglycerol and
80 to 20% by weight fatty acid diester of the polyglycerol with a content of 15 to 0% by weight triesters and higher esters of the polyglycerol and a content of more than 40% C₁₀ and/or C₈ in the ester component of the polyglycerol,
and no quantities to only small quantities of less than 5% by weight of free polyglycerol,
the polyglycerol fatty acid ester mixture having a content of
20 to 45% by weight diglycerol fatty acid ester,
30 to 45% by weight triglycerol fatty acid ester and
50 to 10% by weight tetra- and higher polyglycerol fatty acid esters.

8. A detergent, cleaning agent and/or body-cleansing agent (including hair-cleansing agent) according to Claim 7, characterised in that the polyglycerol fatty acid ester mixture (relative to 100 parts by weight of the polyglycerol fatty acid ester mixture) consists of
35 to 65% by weight fatty acid monoester of the polyglycerol with a content of 50% and more C₁₀ and/or C₈ in the ester component of the polyglycerol and
65 to 35% by weight fatty acid diester of the polyglycerol with a content of 7 to 0.5% by weight triesters and higher esters of the polyglycerol and a content of 50% and more C₁₀ and/or C₈ in the ester component of the polyglycerol,
and no quantities to only small quantities of less than 2.5% by weight of free polyglycerol,
the polyglycerol fatty acid ester mixture having a content of
25 to 42% by weight diglycerol fatty acid ester,
32 to 43% by weight triglycerol fatty acid ester and
43 to 15% by weight tetra- and higher polyglycerol fatty acid esters.

9. A detergent, cleaning agent and/or body-cleansing agent (including hair-cleansing agent) according to Claims 7 and 8, characterised in that the fatty acid esters of the polyglycerol have a content of more than 90% C₁₀ and/or C₈ in the ester group of the polyglycerol.

10. A method for preparing polyglycerol fatty acid esters with one of the compositions given in Claims 1 to 3 from fatty acids with C₈ to C₂₄ and polyglycerol with acid catalysis, characterised in that a mixture which (relative to 100 parts by weight polyglycerols used) consists of
20 to 45% by weight diglycerol,
30 to 45% by weight triglycerol and
50 to 10% by weight tetra- and higher polyglycerols
is used as polyglycerol and is reacted in a vacuum with the fatty acid or the fatty acid mixture
at 140 to 220°C, preferably
150 to 160°C,
in the presence of an acidic catalyst, preferably a compound containing sulphonic acid groups, in a ratio of the polyglycerol mixture to the fatty acid or the fatty acid mixture of
0.5:1.5 to 1.5:0.5, preferably
0.6:1 to 1.2:1,
in the presence of an inert gas, preferably nitrogen, and the resulting reaction product is continuously distilled off until an acid number of < 3 is reached in the reaction mixture, the reaction mixture is cooled to 60 to 115°C, preferably 80 to 110°C, and any polyglycerol contents separating out are removed, whereafter an organic solvent is added to the reaction mixture, containing non-reacted polyglycerol contents at 0 to 100°C, preferably at 20 to 80°C, and non-reacted contents of polyglycerol are extracted in at least one extraction stage with subsequently-added water, which contains a neutralising agent, preferably in approximately equimolecular quantities by weight to the quantity of catalyst, corresponding to the quantity of catalyst added, and the organic phase separated off is separated from the organic solvent used and the residual water content for preparing the polyglycerol fatty acid ester mixture by distillation.

11. A method according to Claim 10, characterised in that the reaction mixture after cooling to 60 to 115°C, preferably 80 to 110°C, is allowed to stand for more than 0.5 hours, preferably 1 to 10 hours, before the extraction and/or before the further processing, and any contents of polyglycerols which have separated out are separated off.

12. A method according to Claims 10 and 11, characterised in that the reaction mixture, for extraction or removal of the remaining (non-reacted) polyglycerol contents, is mixed with an organic-chemical solvent or solvent mixture which has a water absorption capacity of less than 30% by weight, preferably less than 20% by weight (relative to 100 parts by weight of the organic-chemical solvent or solvent mixture) and/or forms an azeotropic mixture with water upon the distillation or in the gas phase and subsequently the remaining polyglycerol is extracted with water.

13. The use of the polyglycerol fatty acid ester mixture according to Claims 1 to 3 for solubilisation or as an emulsifier, preferably for the solubilisation of ethereal oils and perfume oils in water.

## Revendications

1. Mélange d'esters d'acides gras et de polyglycérol ayant un constituant ester d'acides gras en C₈ à C₂₄, caractérisé en ce que le mélange d'esters d'acides gras et de polyglycérol est constitué, pour 100 parties en poids du mélange d'esters d'acides gras du polyglycérol
de 20 à 80 % en poids de monoester d'acide gras du polyglycérol contenant plus de 40 % de C₁₀ et/ou C₈ dans le constituant ester du polyglycérol, et
de 80 à 20 % en poids de diester d'acide gras du polyglycérol contenant de 15 à 0 % en poids de triester et d'esters supérieurs du polyglycérol et plus de 40 % de C₁₀ et/ou C₈ dans le constituant ester du polyglycérol,
et ne contient pas de polyglycérol libre, ou seulement de faibles quantités de ce dernier, inférieures à 5 % en poids,
le mélange d'esters d'acides gras du polyglycérol contenant :
20 à 45 % en poids d'ester d'acide gras du diglycérol,
20 à 45 % en poids d'ester d'acide gras du triglycérol, et
50 à 10 % en poids d'esters d'acides gras du tétraglycérol et de polyglycérols supérieurs.

2. Mélange d'esters d'acides gras du polyglycérol selon la revendication 1, caractérisé en ce que le mélange d'esters d'acides gras du polyglycérol est constitué, pour 100 parties en poids du mélange d'esters d'acides gras du polyglycérol
de 35 à 65 % en poids de monoester d'acide gras du polyglycérol contenant 50 % et plus de C₁₀ et/ou C₈ dans le constituant ester du polyglycérol, et
de 65 à 35 % en poids de diester d'acide gras du polyglycérol contenant de 7 à 0,5 % en poids de triester et d'esters supérieurs du polyglycérol et 50 % et plus de C₁₀ et/ou C₈ dans le constituant ester du polyglycérol,
et ne contient pas de polyglycérol libre ou n'en contient que de faibles quantités, inférieures à 2,5 % en poids,
où le mélange d'esters d'acides gras du polyglycérol contient
de 25 à 42 % en poids d'ester d'acide gras du diglycérol,
de 32 à 43 % en poids d'ester d'acide gras du triglycérol, et
de 43 à 15 % en poids d'esters d'acides gras du tétraglycérol et de polyglycérols supérieurs.

3. Mélange d'esters d'acides gras du polyglycérol selon les revendications 1 et 2, caractérisé en ce que les esters d'acides gras du polyglycérol contiennent plus de 90 % de C₁₀ et/ou C₈ dans le groupe ester du polyglycérol.

4. Epaississant et/ou additif pour les soins de la peau, contenant un mélange d'esters d'acides gras et de polyglycérol ayant un constituant acide gras en C₈ à C₂₄, caractérisé en ce que le mélange d'esters d'acides gras et de polyglycérol est constitué, pour 100 parties en poids du mélange d'esters d'acides gras du polyglycérol
de 20 à 80 % en poids de monoester d'acide gras du polyglycérol contenant plus de 40 % de C₁₀ et/ou C₈ dans le constituant ester du polyglycérol, et
de 80 à 20 % en poids d'un diester d'acide gras du polyglycérol contenant de 15 à 0 % en poids de triester et d'esters supérieurs du polyglycérol et plus de 40 % de C₁₀ et/ou C₈ dans le constituant ester du polyglycérol,
et ne contient pas de polyglycérol libre, ou seulement de faibles quantités de ce dernier, inférieures à 5 % en poids,
le mélange d'esters d'acides gras du polyglycérol contenant :
20 à 45 % en poids d'ester d'acide gras du diglycérol,
30 à 45 % en poids d'ester d'acide gras du triglycérol, et
50 à 10 % en poids d'esters d'acides gras du tétraglycérol et de polyglycérols supérieurs.

5. Epaississant et/ou additif pour les soins de la peau selon la revendication 4, caractérisé en ce que le mélange d'esters d'acides gras du polyglycérol est constitué, pour 100 parties en poids du mélange d'esters d'acides gras du polyglycérol
de 35 à 65 % en poids de monoester d'acide gras du polyglycérol contenant 50 % et plus de C₁₀ et/ou C₈ dans le constituant ester du polyglycérol, et
de 65 à 35 % en poids de diester d'acide gras du polyglycérol contenant de 7 à 0,5 % en poids de triester et d'esters supérieurs du polyglycérol et 50 % et plus de C₁₀ et/ou C₈ dans le constituant ester du polyglycérol,
et ne contient pas de polyglycérol libre ou n'en contient que de faibles quantités, inférieures à 2,5 % en poids,
où le mélange d'esters d'acides gras du polyglycérol contient
de 25 à 42 % en poids d'ester d'acide gras du diglycérol,
de 32 à 43 % en poids d'ester d'acide gras du triglycérol, et
de 43 à 15 % en poids d'esters d'acides gras du tétraglycérol et de polyglycérols supérieurs.

6. Epaississant et/ou additif pour les soins de la peau selon les revendications 4 et 5, caractérisé en ce que l'ester d'acide gras du polyglycérol contient plus de 90 % de C₁₀ et/ou de C₈ dans le groupe ester du polyglycérol.

7. Produit de lavage, de nettoyage et/ou de nettoyage corporel (et notamment produit pour le nettoyage des cheveux), contenant un mélange d'esters d'acides gras du polyglycérol ayant un constituant ester d'acide gras en C₈ à C₂₄ et au moins un tensioactif ionique et/ou amphotère, caractérisé en ce que le mélange d'esters d'acides gras et de polyglycérol est constitué, pour 100 parties en poids du mélange d'esters d'acides gras du polyglycérol
de 20 à 80 % en poids de monoester d'acide gras du polyglycérol contenant plus de 40 % de C₁₀ et/ou C₈ dans le constituant ester du polyglycérol, et
de 80 à 20 % en poids de diester d'acide gras du polyglycérol contenant de 15 à 0 % en poids de triester et d'esters supérieurs du polyglycérol et plus de 40 % de C₁₀ et/ou C₈ dans le constituant ester du polyglycérol,
et ne contient pas de polyglycérol libre, ou seulement de faibles quantités de ce dernier, inférieures à 5 % en poids,
le mélange d'esters d'acides gras du polyglycérol contenant :
20 à 45 % en poids d'ester d'acide gras du diglycérol,
30 à 45 % en poids d'ester d'acide gras du triglycérol, et
50 à 10 % en poids d'esters d'acides gras du tétraglycérol et de polyglycérols supérieurs.

8. Produit de lavage, de nettoyage et/ou de nettoyage corporel (et notamment produit pour le nettoyage des cheveux) selon la revendication 7, caractérisé en ce que le mélange d'esters d'acides gras du polyglycérol est constitué, pour 100 parties en poids du mélange d'esters d'acides gras du polyglycérol
de 35 à 65 % en poids de monoester d'acide gras du polyglycérol contenant 50 % et plus de C₁₀ et/ou C₈ dans le constituant ester du polyglycérol, et
de 65 à 35 % en poids de diester d'acide gras du polyglycérol contenant de 7 à 0,5 % en poids d'un triester et d'esters supérieurs du polyglycérol et 50 % et plus de C₁₀ et/ou C₈ dans le constituant ester du polyglycérol,
et ne contient pas de polyglycérol libre ou n'en contient que de faibles quantités, inférieures à 2,5 % en poids,
où le mélange d'esters d'acides gras du polyglycérol contient
de 25 à 42 % en poids d'ester d'acide gras du diglycérol,
de 32 à 43 % en poids d'ester d'acide gras du triglycérol, et
de 43 à 15 % en poids d'esters d'acides gras du tétraglycérol et de polyglycérols supérieurs.

9. Produit de lavage, de nettoyage et/ou de nettoyage corporel (et notamment produit pour le nettoyage des cheveux) selon les revendications 7 et 8, caractérisé en ce que les esters d'acides gras du polyglycérol contiennent plus de 90 % de C₁₀ et/ou de C₈ dans le groupe ester du polyglycérol.

10. Procédé pour préparer des esters d'acides gras du polyglycérol ayant la composition donnée dans les revendications 1 à 3, à partir d'acides gras en C₈ à C₂₄ et de polyglycérol sous catalyse acide, caractérisé en ce que l'on utilise comme polyglycérol un mélange qui est constitué, pour 100 parties en poids de polyglycérols utilisés,
de 20 à 45 % en poids de diglycérol,
de 30 à 45 % en poids de triglycérol, et
de 50 à 10 % en poids de tétraglycérols et de polyglycérols supérieurs,
ce mélange étant mis à réagir sous vide avec l'acide gras ou le mélange d'acides gras
à une température de 140 à 220°C, de préférence de 150 à 160°C,
en présence d'un catalyseur acide, de préférence un composé sulfoné, selon un rapport du mélange de polyglycérols à l'acide gras ou au mélange d'acides gras
de 0,5:1,5 à 1,5:0,5 et de préférence de 0,6:1 à 1,2:1,
en présence d'un gaz inerte, de préférence l'azote, le produit de réaction qui se forme étant chassé en continu par distillation, et ce jusqu'à ce que l'on arrive dans le mélange réactionnel à un indice d'acide < 3 ; on refroidit le mélange réactionnel à une température de 60 à 115°C et de préférence de 80 à 110°C, et on élimine éventuellement les fractions de polyglycérol qui se séparent ; ce après quoi on ajoute un solvant organique au mélange réactionnel, qui contient des fractions polyglycérol intactes, à une température de 0 à 100°C et de préférence de 20 à 80°C ; et les fractions intactes de polyglycérol sont extraites dans au moins un étage d'extraction, avec de l'eau ajoutée après-coup, qui contient, selon la quantité ajoutée de catalyseur, un agent de neutralisation, de préférence en des quantités pondérales approximativement équimolaires par rapport à la quantité de catalyseur ; et, pour préparer le mélange d'esters d'acides gras du polyglycérol, on sépare par distillation la phase organique séparée du solvant organique utilisé et de l'eau résiduelle.

11. Procédé selon la revendication 10, caractérisé en ce que le mélange réactionnel est, après refroidissement à une température de 60 à 115°C et de préférence de 80 à 110°C, abandonné avant extraction et/ou avant posttransformation pendant plus de 0,5 heure, de préférence pendant 1 à 10 heures, les fractions éventuellement précipitées étant séparées des polyglycérols.

12. Procédé selon les revendications 10 et 11, caractérisé en ce qu'on ajoute au mélange réactionnel, pour extraire ou éliminer les fractions polyglycérol résiduelles (intactes), un solvant de la chimie organique ou un mélange de solvants de la chimie organique, qui présente un pouvoir d'absorption de l'eau inférieur à 30 % en poids et de préférence inférieur à 20 % en poids (pour 100 parties en poids du solvant ou du mélange de solvants de la chimie organique) et/ou qui forme avec l'eau, lors de la distillation ou en phase gazeuse, un mélange azéotropique, puis on extrait à l'eau le polyglycérol résiduel.

13. Utilisation du mélange d'esters d'acides gras du polyglycérol selon les revendications 1 à 3 pour assurer une solubilisation ou comme émulsifiant, de préférence pour solubiliser des huiles essentielles et des huiles de parfum dans l'eau.
